# EUROPEAN PATENT APPLICATION

(11) **EP 4 603 482 A1**
(43) Date of publication of application: **20.08.2025**
(21) Application number: 23876719.8
(22) Date of filing: 11.10.2023
(51) Int. Cl.: C07D 319/12, C08G 63/08

(54) **METHOD FOR PURIFYING CRYSTAL SUBSTANCE**

(30) Priority: 12.10.2022 CN 202211247973
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: XIONG, Wentao, Shanghai 201208 (CN); WANG, Rui, Shanghai 201208 (CN); ZHOU, Fen, Shanghai 201208 (CN); YANG, Rundong, Shanghai 201208 (CN)
(74) Representative: karo IP
(86) International application number: PCT/CN2023/123900
(87) International publication number: WO 2024/078518

(57) **Abstract**

The present invention provides a phase transition-diffusion coupled crystallization method for purifying a crystalline substance, comprising the following steps: (1) adding a crude crystalline substance in molten state to a solid-liquid mixture I in dissolution equilibrium state to obtain a solid-liquid mixture II; wherein the solid-liquid mixtures I and II each comprise a solid phase and a solution phase, the solid phase comprises the crystalline substance in solid state and possible impurities, and the solution phase comprises a solvent, the crystalline substance in dissolved state and possible impurities; and (2) subjecting the solid-liquid mixture II to a solid-liquid separation.

## Description

### TECHNICAL FIELD

The present invention relates to a method for purifying a crystalline substance (such as glycolide).

### BACKGROUND ART

Ring-opening polymerization is an important chemical reaction process, and its application value has been fully reflected in many fields. At present, with the development of industrial catalysis and polymerization technology, the applicability of ring-opening polymerization is constantly expanding, so that more types of cyclic monomers can be used in the synthesis of polymer materials. Ring-opening polymerization is one of the key steps in the synthesis of polymer materials. For example, the preparation of biodegradable polymers such as polyglycolic acid (PGA), polylactic acid (PLA) and polycaprolactone (PCL) all rely on ring-opening polymerization. These polymer materials have a wide range of applications in the medical, packaging and biomedical fields, and have a positive impact on environmental protection. They can be used to synthesize important compounds in the biomedical field, such as drug carriers, biosensors and drug molecules. These applications contribute to improving drug delivery and therapeutic effects.

Cyclic monomers are usually used as raw materials in ring-opening polymerization reactions. These cyclic monomers usually have reactive groups that are easy to open the ring, such as esters, ethers, ketones, etc., and can be used to prepare corresponding linear polymers under certain conditions and the function of catalysts. However, the above-mentioned cyclic monomers usually contain trace impurities from the upstream monomer synthesis unit. These impurities may originate from the synthesis process of the raw materials, separation and purification process, and undesirable storage conditions. Common trace impurities that may exist in cyclic monomers include unreacted raw materials (during the process of synthesizing cyclic monomers, incompletely reacted starting monomers or reaction intermediates may remain), by-products (the reaction of synthesizing cyclic monomers may produce by-products, which are usually unwanted), catalyst residues, solvent residues (during the process of synthesizing and separating cyclic monomers, solvent(s) is/are usually used to assist the reaction and separation. If the solvent(s) is/are not completely removed, they may also become the source of impurities), oxidation products (cyclic monomers may be oxidized by oxygen, resulting in the formation of oxidation products), and accidental ring-opening (cyclic monomers may be affected by small molecules such as water, resulting in the formation of ring-opening products such as hydrolysates).

Removal of these impurities is very important because they may negatively affect the efficiency of the ring-opening polymerization reaction and the quality of the product. Through the necessary purification and separation steps, the presence of these impurities can be reduced to ensure product quality, improve reaction efficiency, meet environmental protection requirements and ensure application safety. In actual production, the refining of ring-opening polymerization monomers is crucial for the successful realization of the target application of the material.

Taking the purification of glycolide, the polymerization monomer of polyglycolic acid (PGA), as an example, the glycolide obtained in industry usually contains various impurities such as water, glycolic acid, glycolic acid oligomers, etc. and is called crude glycolide. The very small amount of active hydrogen contained in these impurities will have a great impact on the molecular weight of the polymer during the ring-opening polymerization of glycolide. Therefore, the crude glycolide obtained from the upstream device (such as the depolymerization ring-forming unit) needs to be purified by recrystallization or washing, etc., which is generally called refining treatment.

The reported methods for refining glycolide include multiple recrystallizations using various organic solvents. For example, in Example 3 of the U.S. invention patent US4727163A, crude glycolide obtained by depolymerization of glycolic acid oligomers was refined by using ethyl acetate. The crude glycolide can only be purified to 90% purity with a yield of 59.4% by two recrystallizations. In order to obtain glycolide with higher purity, more recrystallizations need to be implemented, resulting in a higher solvent usage and a large amount of waste solvent to be treated with various impurities in the crude glycolide contained therein. Also, the recrystallization operation is usually complicated. Therefore, many researchers are exploring new methods for purifying glycolide.

There are also glycolide refining schemes with multiple alcohol washings in the prior art. Chinese invention patent CN107868075A discloses a method for refining glycolide, wherein crude glycolide is purified by using a scheme of cooling recrystallization, mixing and washing with a poor solvent followed by filtering and drying. Alcohols are used as washing solvents in this method. The solubility of glycolide in alcohols is significantly lower than that in good solvents such as ethyl acetate, thereby improving the single-pass refining yield. However, the amount of solvents used in the purification is still high, and there is also the problem of high difficulty in solvent recycling.

Japanese invention patent JP1984148777A discloses a method for purifying crude glycolide by receiving crude glycolide melt with ethyl acetate at lower temperature, which can avoid thermal deterioration of crude glycolide and obtain high-purity glycolide with a higher yield. At a relatively low temperature, crude glycolide melted by heating is added dropwise to an organic solvent under stirring, and then the resulting glycolide suspension is cooled to a specific temperature, and the precipitate is separated and dried. However, this method suffers from the problem of competition between the dissolution of the melt in the organic solvent and the cooling crystallization, resulting in problems such as uncertainty in the crystal precipitation position and precipitation time, thereby causing problems such as uneven particle diameter of precipitated crystals and low extraction efficiency.

### SUMMARY OF INVENTION

In order to solve the technical problems existing in the prior art, the present invention provides a method for purifying a crystalline substance, which can be particularly used for purifying glycolide.

The present invention provides a phase transition-diffusion coupled crystallization method for purifying a crystalline substance, comprising the following steps:
(1) adding a crude crystalline substance in molten state to a solid-liquid mixture I in dissolution equilibrium state to obtain a solid-liquid mixture II; wherein the solid-liquid mixtures I and II each comprise a solid phase and a solution phase, the solid phase comprises the crystalline substance in solid state and possible impurities, and the solution phase comprises a solvent, the crystalline substance in dissolved state and possible impurities; and
(2) subjecting the solid-liquid mixture II to a solid-liquid separation.

In the present invention, preferably, the crude crystalline substance in molten state has a temperature higher than that of the solid-liquid mixture I, and the temperature difference thereof is sufficient to cause the crude crystalline substance in molten state to undergo a phase transition and complete the phase transition.

In the present invention, preferably, the crystalline substance in the solid phase of the solid-liquid mixture I has a purity higher than that of the crude crystalline substance in molten state, and the purity difference thereof is sufficient to cause an effective amount of impurities in the crude crystalline substance in molten state to diffuse into the solvent.

In the present invention, preferably, an equipment containing the solid-liquid mixture I in step (1) is equipped with a heat exchange device to remove the heat brought by the addition of the crude crystalline substance in molten state, such as a jacket or a coil, in which a cooling medium such as water is used.

A typical example of the crystalline substance described in the present invention is glycolide. In this case, according to the present invention, a crude glycolide melt is added into the solid-liquid mixture I at a certain rate, thereby simultaneously performing cooling crystallization and extraction processes. That is, the crude glycolide melt precipitates rapidly and uniformly as solid particles, while at the same time, impurities are extracted into the liquid phase to generate purified glycolide crystals, which can then be recovered through the solid-liquid separation process.

When the crystalline substance is glycolide, the present invention solves several problems existing in the prior art by adding a certain amount of solid crystal particles into a pure organic solvent and using the solid-liquid mixture to receive the glycolide melt. Compared with the traditional recrystallization process, the present invention enables a constant process temperature and no cooling process with a large temperature span, and has the advantages of simple operation, easy industrial scale-up and low energy consumption.

In the prior art, the problem of high acid content is solved by increasing the amount of organic solvent and the number of refining stages, which brings about the problems of high solvent usage, long refining process, and complicated operation. In the purification schemes in which no organic solvent is used, such as melt crystallization and distillation, the problem of high impurity content is also solved by increasing the number of refining stages, which also suffers from the problems of long refining process and complicated operation. The present invention not only solves the technical problem of high acid content of crystalline substances (such as glycolide), but also solves the problem of uneven particle diameter of precipitated crystals during the crystallization process, and provides a simple and efficient refining treatment method for crystalline substances (such as glycolide), and the purified crystalline substances (such as glycolide) have uniform particle diameter and low impurity content.

### DETAILED DESCRIPTION OF INVENTION

In the present invention, the "crude crystalline substance" refers to the substance to be purified by the method of the present invention, which has a relatively low purity, which needs to be improved by the method of the present invention. As an example, the crude crystalline substance has a purity of about 90% or less. Accordingly, an example of the concentration of "to be purified" in the present invention may refer to a purity of about 90% or less.

According to the present invention, the solid-liquid mixture I and solid-liquid mixture II are solid-liquid mixtures each containing the crystalline substance in solid state and the solution of the crystalline substance before and after the addition of the crude crystalline substance in step (1) of the method of the present invention, and they have relatively stable compositions, respectively. In contrast, it should be understood that there is still a solid-liquid mixture during the transformation of the solid-liquid mixture I to the solid-liquid mixture II, and the solid-liquid mixture can be referred to as an "intermediate solid-liquid mixture" in the present invention. As step (1) proceeds, the composition of the intermediate solid-liquid mixture gradually changes from solid-liquid mixture I to solid-liquid mixture II.

According to the present invention, there is no particular limitation on the preparation of the solid-liquid mixture I, as long as the crystalline substance can be in dissolution equilibrium state. For example, the solid-liquid mixture I described in the present invention can be obtained by adding the crystalline substance in solid or molten state with a certain purity to a solvent and mixing thoroughly until it is in dissolution equilibrium.

In the present invention, it can be considered that three different phases are involved, namely, a melt phase (crude crystalline substance in molten state), a solution phase and a solid phase. It should be understood that each phase contains not only the crystalline substance to be purified or purified, but also impurities as appropriate. In other words, the melt phase in the present invention comprises the crystalline substance in molten state and possible impurities; the solid phase comprises the crystalline substance in solid state and possible impurities; and the solution phase comprises a solvent, and the crystalline substance in dissolved state and possible impurities. It should be understood that, preferably, the "crystalline substance" mentioned in the three different phases of the present invention are chemically regarded as the same substance.

According to the present invention, which is different from traditional melt crystallization or solution crystallization, the crystalline substance in molten state is added to the solid-liquid mixture I of the crystalline substance in supersaturated state, so as to utilize the phase transition-diffusion coupled effect of three states of crystalline substance (molten state, dissolved state and solid state) under specific conditions during the crystallization process; wherein, one or both of the crystalline substance in molten state and the solution of the crystalline substance can be the crystalline substance with a purity to be purified, while the crystalline substance in solid state has a higher purity.

According to melt crystallization, impurities are removed through the solid-liquid equilibrium of the melting and crystallization of the substances to be separated themselves without adding a solvent. It is achieved by gradually lowering the temperature of the initial liquid state to achieve partial crystallization based on the different freezing points of the substances to be separated.

The present invention is completely different. Although according to the present invention, the crude crystalline substance in molten state is added to the solid-liquid mixture I of the crystalline substance in supersaturated state and a phase transition of the crude crystalline substance in molten state is involved, the crude crystalline substance in molten state is not subjected to a gradual decrease in temperature during the process. On the contrary, in the specific phase transition-diffusion coupled crystallization method of the present invention, since the crude crystalline substance in molten state is added to the solid-liquid mixture I in a controlled manner and rate, and the temperature of the solid-liquid mixture remains unchanged or substantially unchanged during the process, the crude crystalline substance in molten state completes the phase transition microscopically within seconds.

On the other hand, according to solution crystallization, solid substances are purified based on the different solubility of substances at different temperatures. This method is mainly used for substances whose solubility decreases significantly as the temperature drops. Generally, the substance to be purified is dissolved in a good solvent. The conventional operation of solution crystallization is to lower the temperature of a saturated solution with a relatively high temperature to make it supersaturated and precipitate crystals.

The present invention is completely different. Although according to the present invention, a solution of the crystalline substance to be purified is used, the present invention actually utilizes the solid-liquid mixture I in dissolution equilibrium state between a saturated solution of the crystalline substance and its undissolved solid.

Also, unlike conventional solution crystallization, which generally uses a good solvent for the crystalline substance to be purified, the present invention has no such requirement, and in some embodiments, it is preferred to use a poor solvent for the crystalline substance to be purified; and/or it is preferred that the solvent used is a good solvent for the impurities contained in the crystalline substance to be purified. For the purpose of the present invention only, "good solvent" and "poor solvent" have meanings consistent with the general understanding of the art. According to a preferred embodiment of the present invention, the solvent used is a poor solvent for the crystalline substance to be purified, for example, the solubility of the crystalline substance to be purified in per 100 grams of solvent is less than or equal to 10 grams; on the other hand, it is also preferred that the solubility of the crystalline substance to be purified in per 100 grams of solvent is more than or equal to 0.1 grams, preferably more than or equal to 0.2 grams. Thus, according to a preferred embodiment of the present invention, the solvent used is a good solvent for the impurities contained in the crystalline substance to be purified, and is also a poor solvent for the crystalline substance to be purified.

In addition, unlike the temperature drop during the process of solution crystallization, during the implementation of the method of the present invention, the temperature of the solid-liquid mixture I remains unchanged or substantially unchanged.

Different from the solution crystallization method, which mainly utilizes the precipitation and crystallization of the substance to be purified from the solution, in the specific phase transition-diffusion coupled crystallization method of the present invention, one of the important reasons why the substance to be purified can be purified is that during step (1), the impurities in the system diffuse into the solid-liquid mixture I and then into the intermediate solid-liquid mixture. In particular, in the phase transition-diffusion coupled crystallization method of the present invention, the phase transition of the crude crystalline substance in molten state and the diffusion of the impurities are carried out simultaneously or substantially simultaneously. In other words, for example, the diffusion of impurities occurs simultaneously during most (e.g., more than half) of the phase transition process, until the diffusion equilibrium is reached in the system and the impurity concentration in the solution no longer changes, thereby obtaining the solid-liquid mixture II.

In the phase transition-diffusion coupled crystallization method of the present invention, for the phase transition of the crude crystalline substance in molten state, it is preferred that there is a sufficient temperature difference between the crude crystalline substance in molten state and the solid-liquid mixture I, for example, the temperature difference is more than or equal to 15°C, preferably more than or equal to 25°C, so as to provide sufficient driving force for the phase transition. At the same time, the method of the present invention is not a separate phase transition process, but a phase transition-diffusion coupled process. Therefore, considering the diffusion process (especially the diffusion of impurities) and the dissolution of impurities in the solvent, it is preferred that the temperature difference is less than or equal to 85°C, preferably less than or equal to 75°C.

In the phase transition-diffusion coupled crystallization method of the present invention, the driving force for the phase transition of the crude crystalline substance in molten state is the temperature difference; or more strictly speaking, the solid-liquid mixture I provides sufficient cooling capacity for the crude crystalline substance in molten state. At the same time, without control, the addition of the crude crystalline substance in molten state to the solid-liquid mixture I will cause the temperature of the intermediate solid-liquid mixture obtained in the system to gradually increase; therefore, in order to continuously provide sufficient driving force for the phase transition, it is preferred to control the temperature of the system (especially the intermediate solid-liquid mixture) so that the temperature during the transformation of the solid-liquid mixture I to the solid-liquid mixture II remains unchanged or substantially unchanged; changes by no more than 15°C, preferably by no more than 10°C. For example, the equipment containing the solid-liquid mixture I in step (1) is equipped with a heat exchange device to remove the heat brought by the addition of the crude crystalline substance in molten state, such as a jacket or a coil, in which a cooling medium such as water is used.

It is easy to understand that for a given crude crystalline substance to be purified, obtaining a sufficient temperature difference between it and the solid-liquid mixture can increase the temperature of the crude crystalline substance in molten state, or reduce the temperature of the solid-liquid mixture I. However, similarly, the method of the present invention is not a separate phase transition process, but a phase transition-diffusion coupled process. Therefore, considering the diffusion process (especially the diffusion of impurities) and the dissolution of impurities in the solvent, it is preferred that the temperature of the solid-liquid mixture I, the intermediate solid-liquid mixture and/or the solid-liquid mixture II is not too low, for example, more than or equal to 4°C.

As mentioned above, in the phase transition-diffusion coupled crystallization method of the present invention, the driving force for the phase transition of the crude crystalline substance in molten state is the temperature difference; therefore, it is preferred to gradually and controllably add the crude crystalline substance in molten state to the solid-liquid mixture I in batches or continuously, so that the added crude crystalline substance in molten state obtains sufficient driving force for phase transition. Accordingly, it is preferred that the solid-liquid mixture I is added to the solid-liquid mixture II in a manner selected from at least one of dropwise addition, injection, spraying, sprinkling, atomization.

As an example, the crude crystalline substance in molten state is added to the solid-liquid mixture I at a certain rate. Since the crude crystalline substance in molten state changes into solids, the content of the suspended solid phase in the intermediate solid-liquid mixture gradually increases with the addition of the crude crystalline substance in molten state. This process can be referred to as a feeding process 1 for the purpose of the present invention. In this process 1, no raw materials are removed from the system, and no materials other than the crude crystalline substance in molten state are added to the system, while the amount of the intermediate solid-liquid mixture gradually increases.

Alternatively, after the feeding process 1 is finished (for example, diffusion equilibrium is reached in the system), the crude crystalline substance in molten state can continue to be added to the dynamically configured solid-liquid mixture at a certain rate to stabilize the content of the suspended solid phase in the solid-liquid mixture II and to achieve continuous operation; this process can be referred to as feeding process 2 for the purpose of the present invention. In this feeding process 2, a solvent having the same temperature as the solid-liquid mixture, which is the same as the solvent used in the solid-liquid mixture, is added to the solid-liquid mixture II while the crude crystalline substance in molten state is added. Due to the addition of the solvent, the content of the suspended solid phase in the solid-liquid mixture II can be stabilized. At the same time, the solid-liquid mixture is discharged from the equipment at a fixed rate, and the discharge flow rate is equal to the sum of the addition flow rate of the crude crystalline substance in molten state and the solvent, so that the amount of materials in the equipment can be (substantially) constant.

The phase transition-diffusion coupled crystallization method of the present invention involves the coupling of the phase transition process and the diffusion process, wherein three states of the crystalline substance to be purified are utilized, namely, the molten state, the dissolved state and the solid state. Without being bound by any known theory, it is believed that during the implementation of the method of the present invention, state transformations occur among the three states of the crystalline substance to be purified. However, from a macroscopic perspective of the process and results of the method of the present invention, in general, it is basically equivalent to the crude crystalline substance in molten state added to the solid-liquid mixture I undergoing a phase transition to form the crystalline substance in solid state with improved purity in the solid-liquid mixture II. Therefore, the content of the solid phase in the solid-liquid mixture I (and preferably even the solid-liquid mixture II) of the present invention needs to be under proper control for the purpose of the present invention. Accordingly, the weight content of the solid phase in the solid-liquid mixture I is 0.5%-25%; preferably 2%-25%; and/or, the weight content of the solid phase in the solid-liquid mixture II is less than or equal to 50%; preferably less than or equal to 40%.

Preferably, for the phase transition-diffusion coupled crystallization method of the present invention, in order to facilitate the phase transition process and the diffusion process, the solid phase in the solid-liquid mixture I and/or solid-liquid mixture II also exhibits desirable uniformity.

On one hand, the particles of the solid phase are uniformly distributed in the solid-liquid mixture I and/or the solid-liquid mixture II. For example, when two different samples of the solid-liquid mixture are taken from any two different positions in the solid-liquid mixture I to measure D50 particle diameter, the deviation in D50 particle diameter between the two different samples of the solid-liquid mixture is no more than 30%, preferably no more than 20%; and/or, when two different samples of the solid-liquid mixture are taken from any two different positions in the solid-liquid mixture II to measure D50 particle diameter, the deviation in D50 particle diameter between the two different samples of the solid-liquid mixture is no more than 30%, preferably no more than 20%. Accordingly, for example, the solid-liquid mixture I and/or the solid-liquid mixture II may be a suspension.

On the other hand, the sizes of the individual particles of the solid phase distributed in the solid-liquid mixture I and/or the solid-liquid mixture II are relatively uniform. For example, in the solid phase of the solid-liquid mixture I and/or the solid-liquid mixture II, the ratio of D90 to D10 of the solid phase is respectively no more than 10, preferably no more than 7, and more preferably no more than 5.

As mentioned above, in the phase transition-diffusion coupled crystallization method of the present invention, from a macroscopic observation of the process and results of the method of the present invention, in general, on one hand, the method of the present invention is basically equivalent to the crude crystalline substance in molten state added to the solid-liquid mixture I undergoing a phase transition to form the crystalline substance in solid state with improved purity in the solid-liquid mixture II; on the other hand, the method of the present invention is otherwise basically equivalent to the impurities in the crude crystalline substance in molten state added to the solid-liquid mixture I diffusing into the solution phase of the solid-liquid mixture II. For the purpose of the present invention, the main driving force for phase transition may be temperature difference, while the main driving force for diffusion may be purity difference. However, without being bound by any known theory, it should be understood by those skilled in the art that since the present invention utilizes the phase transition-diffusion coupled effects of the three states of the crystalline substance (molten state, dissolved state and solid state) under specific conditions during the crystallization process, the process actually involved in the present invention is far more complicated than the macroscopic observation, and thus can and should be described as a phase transition-diffusion coupled process.

Thus, for the purpose of the present invention, in one case, it is preferred that the purity of the crystalline substance in the solid phase of the solid-liquid mixture I is higher than that of the crude crystalline substance in molten state, and the purity difference thereof is sufficient to cause an effective amount of impurities in the crude crystalline substance in molten state to diffuse into the solvent. As an example, the purity of the crude crystalline substance in molten state is about 90% or less, for example, 85-90%; while the purity of the crystalline substance in the solid phase of the solid-liquid mixture I is 95% or more, preferably 99% or more, for example, 99.5% or more. In this case, the purity difference (i.e., the impurity content difference) between the crude crystalline substance in molten state and the solid phase of the solid-liquid mixture I may function as an additional driving force for the purification of the crude crystalline substance in molten state. At the same time, for the purpose of the present invention, although one or both of the crude crystalline substance in molten state and the solution of the crystalline substance may contain the crystalline substance with the purity to be purified, in some cases, it is preferred that the solution of the crystalline substance contains the crystalline substance with higher purity. For example, the purity of the crystalline substance in the solution of the crystalline substance is 90% or more, 95% or more, preferably 99% or more.

It is to be understood that the object to be purified of the present invention is mainly the crystalline substance, and thus in some embodiments, phase transition may function as the main driving force of the method of the present invention. Therefore, in some cases, for example, when a crystalline substance with very high purity is desired, the method may be implemented with phase transition as the main driving force starting from a crude crystalline substance that already has a relatively high purity. Accordingly, for example, in some embodiments, the purity of the crude crystalline substance in molten state is about 90% or more, for example, 90-99%.

Thus, for the method of the present invention, the crude crystalline substance with a wide range of purity can be treated. For example, the purity of the crude crystalline substance in molten state (e.g., glycolide by mass) may be 85%-99%.

Accordingly, it should be understood by those skilled in the art that, due to the presence of dual driving forces, i.e., temperature difference and phase transition, crude crystalline substances with lower purity can undergo multiple purifications by repeating the method of the present invention until a desired purity is achieved, for example, a crude crystalline substance with a purity of 85-90% is purified into a crystalline substance with a purity of up to 99% or even higher. As required, the multiple purifications may be implemented for example 2, 3, 4 or 5 times, until a desired purity is achieved. In this case, each time the purification is performed, the purity of the crystalline substance in the solid phase of the solid-liquid mixture I may be the same or different, but preferably before the last purification is performed, the purity of the crystalline substance in the solid phase of the solid-liquid mixture I is higher than that in the crude crystalline substance in molten state.

When the purification method of the present invention is implemented repeatedly, the purified crystalline substance obtained from the previous implementation of the method is melted to serve as the crude crystalline substance in molten state for the next implementation of the method. For example, typically, the method of the present invention can be implemented in a two-step purification mode, wherein the purified crystalline substance obtained from the first implementation of the method is melted to serve as the crude crystalline substance in molten state for the second implementation of the method, wherein preferably, the purity of the crystalline substance in the solid phase of the solid-liquid mixture I used for the second purification is still higher than that in the crude crystalline substance in molten state used for the second purification.

The purification method of the present invention is widely applicable to a variety of specific crystalline substances. In particular, based on the characteristics of the present invention described above, it can be understood that the purification method of the present invention is particularly suitable for a crystalline substance that is distilled in the form of gas phase during the synthesis process, where good solvent(s) exist for the main impurities in this substance, and the crystalline substance product is solid at room temperature. For example, preferably, the crystalline substance is a cyclic monomer with a melting point higher than -20°C, preferably selected from lactones (such as caprolactone, glycolide, lactide, 1,4-dioxane-2-one), cyclic formals (such as trioxane), and lactams (such as caprolactam), preferably lactide compounds, such as glycolide or lactide.

In one embodiment, one of the objects of the present invention is to provide a method for purifying glycolide, comprising the following steps:
(1) adding crude glycolide in molten state to a solid-liquid mixture I, to obtain a solid-liquid mixture II; wherein the solid-liquid mixture I comprises glycolide crystals and a solution of glycolide;
(2) subjecting the solid-liquid mixture II to a solid-liquid separation, and recovering the solid phase to obtain purified glycolide;

Optionally, (3) drying the solid phase recovered in step (2).

In a preferred embodiment of the present invention, in step (1):
the adding manner is selected from at least one of dropwise addition, injection, spraying, sprinkling, atomization; and/or,
the crude glycolide in molten state is unpurified crude glycolide in molten state and/or purified crude glycolide in molten state; and/or,
the weight content of glycolide in the crude glycolide in molten state is 85%-99%.

In a preferred embodiment of the present invention, the unpurified crude glycolide in molten state is obtained by the cracking and ring-forming reaction of glycolic acid oligomers; preferably, it is obtained by condensing a gaseous product generated from the cracking and ring-forming reaction of glycolic acid oligomers, or by deep condensing the gaseous product to obtain solid crude glycolide, which is then heated to melt;

The purified crude glycolide in molten state is a molten substance containing a glycolide phase obtained by purifying crude glycolide, obtained by the cracking and ring-forming reaction of glycolic acid oligomers, using a conventional method. The crude glycolide should be treated into a molten state after being purified by a conventional method and then further purified by the method of the present invention. If the material containing the glycolide phase after being purified by a conventional method is a gas phase, the gas phase should be condensed into a molten state; if the material containing the glycolide phase after being purified by a conventional method is a solid phase, it should be heated to melt into a molten state.

The conventional purification method described above is a conventional purification method for purifying crude glycolide obtained by the cracking and ring-forming reaction of glycolic acid oligomers in the prior art, comprising the steps of extracting, washing, crystallizing, distilling the unpurified crude glycolide.

In a preferred embodiment of the present invention, the temperature of the unpurified crude glycolide in molten state is 75°C-120°C; preferably 80°C-95°C; and/or,
the temperature of the purified crude glycolide in molten state is 80°C-120°C, preferably 85°C-95°C.

For the present invention, the unpurified crude glycolide has a lower melting point. If a mixture of unpurified crude glycolide in molten state and purified crude glycolide in molten state is used, the melting point thereof would increase, and thus the temperature range required to maintain the molten state would increase correspondingly, aligning with that of purified crude glycolide in molten state. Accordingly, for the purpose of the present invention, in view of factors such as different crystalline substances and different purities among crude crystalline substances, the temperature of the crude crystalline substance in molten state that can be used in the present invention only needs to be sufficient to make the crude crystalline substance in molten state. Its temperature is generally near the melting point of the corresponding crystalline substance, but it is not necessarily higher than the melting point of the crystalline substance.

Therefore, in a preferred embodiment of the present invention, the temperature of the crude crystalline substance in molten state is higher than or equal to the melting point of the crystalline substance; preferably, the temperature of the crude crystalline substance in molten state is at least 1°C, preferably at least 3°C higher than the melting point of the crystalline substance, and/or preferably, the temperature of the crude crystalline substance in molten state is no more than 15°C, preferably no more than 10°C higher than the melting point of the crystalline substance.

Alternatively, in a preferred embodiment of the present invention, the temperature of the crude crystalline substance in molten state is lower than or equal to the melting point of the crystalline substance; preferably, the temperature of the crude crystalline substance in molten state is at least 1°C, preferably at least 3°C lower than the melting point of the crystalline substance, and/or preferably, the temperature of the crude crystalline substance in molten state is no more than 15°C, preferably no more than 10°C lower than the melting point of the crystalline substance.

In a preferred embodiment of the present invention, in step (1):
the crude glycolide in molten state is added at a rate of 20 wt%-500 wt%, preferably 50 wt%-200 wt% relative to the weight of the solid-liquid mixture I per hour; the addition rate is expressed as the weight percentage added per unit time relative to the weight of the solid-liquid mixture I;
the temperatures of both the solid-liquid mixture I and the solid-liquid mixture II are not higher than 60°C; preferably, not higher than 50°C; glycolide has the characteristic of susceptible to thermal deterioration, so the solid-liquid mixture I needs to be kept below 60°C before being added.

When the crude glycolide melt is added to the solid-liquid mixture I at a certain rate, the addition process may cause the solid-liquid mixture to heat up due to the higher temperature of the melt itself and the exothermic crystallization upon cooling of the melt. In order to control the temperature of the solid-liquid mixture to be stable, the addition rate needs to be matched with the heat exchange (cooling) power to stabilize the crystallization-extraction process. The temperature of the solid-liquid mixture needs to be kept within the preferred temperature range for two reasons: to avoid thermal deterioration of glycolide and to provide a sufficient temperature difference for the crystallization upon cooling of the glycolide melt. The glycolic acid and glycolic acid oligomers (e.g., glycolic acid dimers, etc.) contained in crude glycolide can provide alcohol hydroxyl groups (initiators) and protonic acids (catalysts) required for the ring-opening polymerization of glycolide. At a certain temperature, for example, a temperature higher than or equal to 60°C, the ring-opening polymerization reaction can occur significantly to generate glycolic acid oligomers with higher molecular weight (polymerization degree more than or equal to 7); the ring-opening polymerization side reaction that occurs during the refining process is a typical thermal deterioration, and its reaction degree is affected by initiator/catalyst concentration, temperature, and time. The generated oligomers are usually difficult to remove due to their low solubility in common organic solvents.

In a preferred embodiment of the present invention, in step (1):
the solid phase of the solid-liquid mixture I is glycolide crystals with a glycolide purity higher than 95%; preferably glycolide crystals with a glycolide purity higher than 99%; and/or,
the liquid phase of the solid-liquid mixture I is a saturated solution of glycolide in an organic solvent; the organic solvent is a good solvent for common impurities in glycolide, such as, at least one of acetone, ethyl acetate, ethanol, n-propanol, iso-propanol, n-butanol, iso-butanol, tert-butanol, n-pentanol, and iso-pentanol. These organic solvents exhibit relatively high solubility for common impurities such as colored impurities and acids in crude glycolide. For example, common acid impurities may be glycolic acid or linear glycolic acid dimers.

In a preferred embodiment of the present invention, in step (1):
the weight content of the solid phase in the solid-liquid mixture I is 0.5%-25%; preferably 2%-25%;
the weight content of the solid phase in the solid-liquid mixture I of more than or equal to 0.5% can provide sufficient crystallization sites for the crystallization upon cooling of the crude glycolide in molten state.

The weight content of the solid phase in the solid-liquid mixture II is less than or equal to 50%, preferably less than or equal to 40%.

In a preferred embodiment of the present invention, in step (1):
the median diameter of the solid phase in the solid-liquid mixture I is less than or equal to 200 microns; preferably less than or equal to 150 microns;
the median diameter of the solid phase in the solid-liquid mixture II is less than or equal to 600 microns; preferably less than or equal to 500 microns;

Relatively small particle diameter of the solid phase in the solid-liquid mixture I is conducive to stirring and dispersion to obtain a uniform solid-liquid mixture;
Conventional means in the prior art may be used to control the particle diameter of the solid phase in the solid-liquid mixture I. For example, the crystal particles may be processed to have the desired particle diameter by physical grinding-screening in advance, and then added to the saturated solution to prepare a mixture. A stirring system with a dispersion function as the main function is preferably used for mixing. In the production equipment, the stirring rate is preferably usually not more than 200 rpm.

Without being bound by any known theory, it is believed that: the particles within the preferred particle diameter range in the solid-liquid mixture I may be uniformly suspended in the solid-liquid mixture under the stirring of conventional stirring paddles such as pitched-blade or helical impellers, and serve as the sites for the crude crystalline substance (such as glycolide) melt to crystallize upon cooling, thereby avoiding the large-scale occurrence of crystallization sites at undesirable positions such as the vessel wall and stirring paddles, and obtaining purified crystalline substance (such as glycolide) crystals with uniform particle diameter; at the same weight content of the solid phase in the solid-liquid mixture I, the smaller the particle diameter of the solid phase, the more particles there are in the solid-liquid mixture, which can provide more crystallization sites, and is beneficial to the uniformity of crystallization; when the crystalline substance (such as glycolide) phase in the crystalline substance (such as crude glycolide) melt contacts the particles in the solid-liquid mixture I, it continues to grow on the surface of the particles, so that the crystal particle diameter in the solid-liquid mixture II is further increased; in order to ensure the efficiency of the extraction process, which enables impurities such as colored impurities and acids to fully diffuse into the organic solvent, for example, enabling more than 80% of the acids to be extracted into the organic solvent, the particle diameter of the glycolide crystals in the solid-liquid mixture II should be as small as possible.

In a preferred embodiment of the present invention, step (3) is implemented, and:
the solid-liquid separation method in step (2) is centrifugation or filtration;
the drying of solid phase in step (3) is convection drying under hot inert gas or thermal conduction drying under vacuum.

In a preferred embodiment of the present invention:
steps (1) and (2) and optionally step (3) are repeated at least once to obtain further purified glycolide crystals.

Another object of the present invention is to provide glycolide obtained by the above purification method.

Another object of the present invention is to provide use of glycolide in the preparation of polyglycolic acid.

The purified glycolide crystals obtained in the present invention can be used as polymerization monomers for polyglycolic acid.

Compared with the prior art, the present invention has the following beneficial effects:
According to the present invention, which is different from the prior art where glycolide is usually added directly into the extraction solvent, a certain amount of solid particles of the crystalline substance (e.g. glycolide crystals) is added into the extraction solvent. The solid particles serve as the sites for the crude crystalline substance (e.g. glycolide) melt to crystallize upon cooling, thereby avoiding the large-scale occurrence of crystallization sites at undesirable positions such as the vessel wall and stirring paddles, and obtaining purified crystalline substance (e.g. glycolide crystals) with uniform particle diameter.

The average particle diameter of the solid phase crystalline substance (such as glycolide crystals) added in the present invention is very small, so that the average particle diameter of the newly generated crystals is relatively small, which is conducive to the sufficient diffusion of impurities such as colored impurities and acids into the organic solvent. This results in higher extraction efficiency and lower acid content in the crystalline substance (such as glycolide). When used for glycolide, compared with the conventional scheme in which the glycolide melt is directly added into the extraction solvent, the method of the present invention achieves lower acid content of glycolide at the same organic solvent usage. Since acidic impurities are the main impurities in crude glycolide, their effective removal enables direct use in ring-opening polymerization. And the purified glycolide exhibits a higher purity.

According to the present invention, a solid-liquid mixture is used to receive the crystalline substance (such as glycolide) melt. Compared with the traditional recrystallization process, the present invention enables a constant process temperature and no cooling process with a large temperature span, and has the advantages of simple operation, easy industrial scale-up and low energy consumption.

### Examples

The present invention is described in detail below in conjunction with specific examples. It is necessary to indicate that the following examples are only used to further illustrate the present invention and cannot be understood as limiting the scope of protection of the present invention. Some non-essential improvements and adjustments made to the present invention by those skilled in the art based on the content of the present invention still fall within the scope of protection of the present invention.

The raw materials used in the examples were all conventional commercially available raw materials.

The average particle diameter of glycolide crystals in the present invention was tested as follows:
Mastersizer 3000 laser particle size analyzer from Malvern company was used to measure the average particle diameter (median diameter, i.e., the particle diameter at which the cumulative volume distribution of particles is 50%), D10 particle diameter (the volume content of particles smaller than this particle diameter accounts for 10% of all particles), and D90 particle diameter (the volume content of particles smaller than this particle diameter accounts for 90% of all particles). The smaller the ratio of D90 to D10, the narrower the particle diameter distribution. Isopropyl alcohol was used as the dispersion medium.

The free acid concentration of crude glycolide and glycolide in the present invention was determined as follows:
The free acid concentration in crude glycolide was determined by an acid-base titration method. The specific operation was performed as follows: the crude glycolide sample was dissolved in about 30 mL of dry dimethyl sulfoxide. After complete dissolution, a few drops of bromophenol blue indicator solution were added, turning the solution yellow. The solution was titrated with a dilute sodium hydroxide solution in benzyl alcohol of known concentration until the color of the solution changed from yellow to green, marking the endpoint. The terminal carboxyl group content in the glycolide (in µmol) was calculated based on the volume of sodium hydroxide solution consumed at the endpoint of titration. This value was divided by the weight of the crude glycolide sample to obtain the free acid concentration of crude glycolide (in µmol/g).

The purity of glycolide in the present invention was determined as follows:
The purity of glycolide was determined by gas chromatography (GC). 200 mg of glycolide sample to be tested and 40 mg of p-chlorobenzophenone as internal standard substance were dissolved in 10 mL of acetone, and 2 µL of the solution was injected into the gas chromatograph to determine the glycolide content; the purity of glycolide was obtained using a standard calibration curve prepared in advance with glycolide reference standards (at least 5 points between 160 and 200 mg) and internal standard substance (40 mg), i.e. p-chlorobenzophenone. The measuring apparatus was Agilent 7890B, the chromatographic column was capillary column HP-5 (30 m × 0.32 mm, 0.25 µm), the chromatographic column temperature was 280°C, the injection port temperature was 150°C, and the detector was FID.

### Example 1

### Preparation of crude glycolide:

600 g of glycolic acid crystals and 6 g of stannous octoate catalyst were added into a reactor. The temperature was raised from room temperature to 90°C until complete dissolution of the solid, then further increased to 120°C for atmospheric prepolymerization. After prepolymerization for 2 h, the temperature was raised to 210°C until no water was distilled out. The system was maintained at the temperature and vacuum was applied with the vacuum degree of this process controlled at 2 kPa(A) until no water was distilled out, yielding 475 g of glycolic acid oligomer.

The oligomer was transferred to a depolymerization reactor. The depolymerization system was reacted at a reaction temperature of 285°C, a vacuum degree of 3 kPa(A), and a stirring rate of 120 rpm to prepare crude glycolide. The crude glycolide vapor produced in the depolymerization reactor was condensed using a spherical condenser with a jacket water temperature of 85°C to obtain crude glycolide in molten state. The reaction was stopped after 2 h, yielding 395 g of crude glycolide with an acid content of 450 µmol/g and a glycolide purity of 90.20%.

### Purification of glycolide:

An excess amount of glycolide solid (commercially available, acid content: 1.7 µmol/g, GC purity: 99.86%) was added to 600 g of ethyl acetate (commercially available, dehydrated with 3A molecular sieve), and the mixture was thoroughly mixed at 10°C until dissolution equilibrium was achieved. The solid-liquid mixture was subjected to solid-liquid separation at 10°C to obtain a clear saturated solution. 100 g of glycolide solid was added to 500 g of the saturated solution so as to obtain a solid-liquid mixture I.

250 g of the above-mentioned crude glycolide melt was maintained at 90°C in jacketed reactor A. 600 g of the solid-liquid mixture I was added to jacketed reactor B, in which 100 g of glycolide solid (with a median diameter of 103 µm, a median diameter deviation (D50 particle diameter deviation) of 9.5% measured by sampling from the upper and lower parts of the reactor) was suspended, and in which the acid content of the liquid phase was 0.15 µmol/g. The mixture was stirred at 120 rpm and maintained at 10°C. The melt from reactor A was added dropwise to reactor B at a rate of 70 wt% relative to the weight of the solid-liquid mixture per hour, during which the stirring rate was 120 rpm and the internal temperature (the temperature of the material in the reactor) was maintained at 12°C. After completion, a solid-liquid mixture with a solid content of 35.3 wt% was obtained. The filter cake was collected by suction filtration under nitrogen protection. The median diameter of the glycolide crystals in the filter cake was measured to be 275 µm (the samples of the solid-liquid mixture were taken from the upper and lower parts of the reactor, the filter cake was collected by suction filtration, and the median diameter of the crystals in the filter cake was measured to have a deviation of 15.0%), and the ratio of D90 to D10 was 3.9. The filter cake was vacuum-dried at 60°C for 4 h to obtain purified glycolide crystals with an acid content of 45 µmol/g and a purity measured by GC of 98.0%.

### Example 2

Crude glycolide was prepared as in Example 1.

### Purification of glycolide:

An excess amount of glycolide solid (commercially available, acid content: 1.7 µmol/g) was added to 600 g of isopropanol (commercially available, dehydrated with 3A molecular sieve), and the mixture was thoroughly mixed at 35°C until dissolution equilibrium was achieved. The solid-liquid mixture was subjected to solid-liquid separation at 35°C to obtain a clear saturated solution. 30 g of glycolide solid was added to 500 g of the saturated solution so as to obtain a solid-liquid mixture I.

250 g of the above-mentioned crude glycolide melt was maintained at 90°C in jacketed reactor A. 530 g of the mixture of isopropanol and glycolide was added to jacketed reactor B, in which 30 g of glycolide solid (with a median diameter of 103 µm, a median diameter deviation of 8.0% measured by sampling from the upper and lower parts of the reactor) was suspended, and in which the acid content of the liquid phase was 0.03 µmol/g. The mixture was stirred at 120 rpm and maintained at 35°C. The melt from reactor A was added dropwise to reactor B at a rate of 70 wt% relative to the weight of the solid-liquid mixture per hour, during which the stirring rate was 120 rpm and the internal temperature was maintained at 36°C. After completion, a solid-liquid mixture with a solid content of 31.7 wt% was obtained. The filter cake was collected by suction filtration under nitrogen protection. The median diameter of the glycolide crystals in the filter cake was measured to be 300 µm (the samples of the solid-liquid mixture were taken from the upper and lower parts of the reactor, the filter cake was collected by suction filtration, and the median diameter of the crystals in the filter cake was measured to have a deviation of 16.1%), and the ratio of D90 to D10 was 4.3. The filter cake was vacuum-dried at 60°C for 4 h to obtain purified glycolide crystals with an acid content of 42 µmol/g and a purity measured by GC of 98.2%.

### Example 3

Crude glycolide was prepared as in Example 1.

### Purification of glycolide:

The saturated solution was prepared as in Example 2. 15 g of glycolide solid was added to 500 g of the saturated solution so as to obtain a solid-liquid mixture I.

250 g of the above-mentioned crude glycolide melt was maintained at 90°C in jacketed reactor A. 515 g of the mixture of isopropanol and glycolide was added to jacketed reactor B, in which 15 g of glycolide solid (with a median diameter of 103 µm, a median diameter deviation of 6.2% measured by sampling from the upper and lower parts of the reactor) was suspended, and in which the acid content of the liquid phase was 0.03 µmol/g. The mixture was stirred at 120 rpm and maintained at 35°C. The melt from reactor A was added dropwise to reactor B at a rate of 70 wt% relative to the weight of the solid-liquid mixture per hour, during which the stirring rate was 120 rpm and the internal temperature was maintained at 36°C. After completion, a solid-liquid mixture with a solid content of 30.4 wt% was obtained. The filter cake was collected by suction filtration under nitrogen protection. The median diameter of the glycolide crystals in the filter cake was measured to be 500 µm (the samples of the solid-liquid mixture were taken from the upper and lower parts of the reactor, the filter cake was collected by suction filtration, and the median diameter of the crystals in the filter cake was measured to have a deviation of 17.9%), and the ratio of D90 to D10 was 4.7. The filter cake was vacuum-dried at 60°C for 4 h to obtain purified glycolide crystals with an acid content of 75 µmol/g and a purity measured by GC of 97.1%.

### Example 4

Crude glycolide was prepared as in Example 1.

### Purification of glycolide:

An excess amount of glycolide solid (commercially available, acid content: 1.7 µmol/g, GC purity: 99.86%) was added to 600 g of ethanol (commercially available, dehydrated with 3A molecular sieve), and the mixture was thoroughly mixed at 45°C until dissolution equilibrium was achieved. The solid-liquid mixture was subjected to solid-liquid separation at 45°C to obtain a clear saturated solution. 75 g of glycolide solid was added to 375 g of the saturated solution so as to obtain a solid-liquid mixture I.

250 g of the above-mentioned crude glycolide melt was maintained at 90°C in jacketed reactor A. 450 g of the mixture of ethanol and glycolide was added to jacketed reactor B, in which 75 g of glycolide solid (with a median diameter of 103 µm, a median diameter deviation of 10.1% measured by sampling from the upper and lower parts of the reactor) was suspended, and in which the acid content of the liquid phase was 0.06 µmol/g. The mixture was stirred at 120 rpm and maintained at 45°C. The melt from reactor A was added dropwise to reactor B at a rate of 70 wt% relative to the weight of the solid-liquid mixture per hour, during which the stirring rate was 120 rpm and the internal temperature was maintained at 48°C. After completion, a solid-liquid mixture with a solid content of 40.0 wt% was obtained. The filter cake was collected by suction filtration under nitrogen protection. The median diameter of the glycolide crystals in the filter cake was measured to be 268 µm (the samples of the solid-liquid mixture were taken from the upper and lower parts of the reactor, the filter cake was collected by suction filtration, and the median diameter of the crystals in the filter cake was measured to have a deviation of 13.2%), and the ratio of D90 to D10 was 5.2. The filter cake was vacuum-dried at 60°C for 4 h to obtain purified glycolide crystals with an acid content of 73 µmol/g and a purity measured by GC of 97.5%.

### Example 5

The purified glycolide crystals (GC purity: 98.0%) obtained in Example 1 were subjected to secondary purification.

### Secondary purification of glycolide:

The saturated solution was prepared as in Example 1. 60 g of glycolide solid was added to 300 g of the saturated solution so as to obtain a solid-liquid mixture I.

150 g of the purified glycolide crystals obtained in Example 1 were heated and melted, and the melt was maintained at 90°C in jacketed reactor A. 360 g of the solid-liquid mixture I of ethyl acetate and glycolide was added to jacketed reactor B, in which 60 g of glycolide solid (with a median diameter of 103 µm, a median diameter deviation of 10.6% measured by sampling from the upper and lower parts of the reactor, and a GC purity of 99.86%) was suspended, and in which the acid content of the liquid phase was 0.14 µmol/g. The mixture was stirred at 120 rpm and maintained at 10°C. The melt from reactor A was added dropwise to reactor B at a rate of 70 wt% relative to the weight of the solid-liquid mixture per hour, during which the stirring rate was 120 rpm and the internal temperature was maintained at 12°C. After completion, a solid-liquid mixture with a solid content of 37.6 wt% was obtained. The filter cake was collected by suction filtration under nitrogen protection. The median diameter of the glycolide crystals in the filter cake was measured to be 295 µm (the samples of the solid-liquid mixture were taken from the upper and lower parts of the reactor, the filter cake was collected by suction filtration, and the median diameter of the crystals in the filter cake was measured to have a deviation of 14.4%), and the ratio of D90 to D10 was 3.6. The filter cake was vacuum-dried at 60°C for 4 h to obtain purified glycolide crystals with an acid content of 2.8 µmol/g and a purity measured by GC of 99.6%.

### Preparation of polyglycolic acid:

55 g of the purified glycolide from Example 5 was weighed and added to a reactor, 0.006% by weight of glycolide of stannous chloride dihydrate powder (commercially available) and 0.1% by weight of glycolide of initiator lauryl alcohol (commercially available) were weighed and added to the reactor, the reactor was evacuated and then replaced with nitrogen, the evacuation-nitrogen replacement operation was repeated once, and finally the reactor was evacuated again to make the pressure inside the reactor lower than 200 Pa(A). Then, the reactor was closed for reaction under stirring. The reactor temperature was first heated to 160°C, and then the temperature was increased at a heating rate of 4°C/min, and finally the temperature was maintained at 220°C, and the reaction was carried out for 60 min before discharging to obtain polyglycolic acid.

The intrinsic viscosity of the polyglycolic acid was measured to be 1.60 dL/g.

### Example 6

The purified glycolide crystals (GC purity: 98.2%) obtained in Example 2 were subjected to secondary purification.

### Secondary purification of glycolide:

The saturated solution was prepared as in Example 2. 18 g of glycolide solid was added to 300 g of the saturated solution so as to obtain a solid-liquid mixture I.

150 g of the purified glycolide crystals obtained in Example 2 were heated and melted, and the melt was maintained at 90°C in jacketed reactor A. 318 g of the mixture of isopropanol and glycolide was added to jacketed reactor B, in which 18 g of glycolide solid (with a median diameter of 103 µm, a median diameter deviation of 8.5% measured by sampling from the upper and lower parts of the reactor, and a GC purity of 99.86%) was suspended, and in which the acid content of the liquid phase was 0.03 µmol/g. The mixture was stirred at 120 rpm and maintained at 35°C. The melt from reactor A was added dropwise to reactor B at a rate of 70 wt% relative to the weight of the solid-liquid mixture per hour, during which the stirring rate was 120 rpm and the internal temperature was maintained at 36°C. After completion, a solid-liquid mixture with a solid content of 34.6 wt% was obtained. The filter cake was collected by suction filtration under nitrogen protection. The median diameter of the glycolide crystals in the filter cake was measured to be 340 µm (the samples of the solid-liquid mixture were taken from the upper and lower parts of the reactor, the filter cake was collected by suction filtration, and the median diameter of the crystals in the filter cake was measured to have a deviation of 15.4%), and the ratio of D90 to D10 was 4.0. The filter cake was vacuum-dried at 60°C for 4 h to obtain purified glycolide crystals with an acid content of 3.2 µmol/g and a purity measured by GC of 99.5%.

### Preparation of polyglycolic acid:

Polyglycolic acid was prepared as in Example 5, except that 55 g of the purified glycolide from Example 6 was weighed and added to the reactor; the intrinsic viscosity of the polyglycolic acid was measured to be 1.58 dL/g.

### Example 7

### Preparation of crude glycolide:

The differences from Example 1 were in that the depolymerization system was reacted at a reaction temperature of 255°C, the crude glycolide vapor produced in the depolymerization reactor was condensed using a spherical condenser with a jacket water temperature of 7°C to obtain crude glycolide in solid state, and the reaction was stopped after 4 h, yielding 372 g of crude glycolide with an acid content of 570 µmol/g and a glycolide purity of 86.0%;

Except for the above-mentioned differences, others were the same as those in Example 1.

### Purification of glycolide:

The solid-liquid mixture I was prepared as in Example 1, except that a different glycolide solid (commercially available, acid content: 6.0 µmol/g, GC purity: 99.30%) was used.

250 g of crude glycolide solid was heated and melted, and the melt was maintained at 93°C in jacketed reactor A. 600 g of the solid-liquid mixture I of ethyl acetate and glycolide was added to jacketed reactor B, in which 100 g of glycolide solid (with a median diameter of 145 µm, a median diameter deviation of 13.0% measured by sampling from the upper and lower parts of the reactor, and a GC purity of 99.30%) was suspended, and in which the acid content of the liquid phase was 0.52 µmol/g. The mixture was stirred at 120 rpm and maintained at 10°C. The melt from reactor A was added dropwise to reactor B at a rate of 50 wt% relative to the weight of the solid-liquid mixture per hour, during which the stirring rate was 120 rpm and the internal temperature was maintained at 12°C. After completion, a solid-liquid mixture with a solid content of 32.5 wt% was obtained. The filter cake was collected by suction filtration under nitrogen protection. The median diameter of the glycolide crystals in the filter cake was measured to be 293 µm (the samples of the solid-liquid mixture were taken from the upper and lower parts of the reactor, the filter cake was collected by suction filtration, and the median diameter of the crystals in the filter cake was measured to have a deviation of 17.5%), and the ratio of D90 to D10 was 3.8. The filter cake was vacuum-dried at 60°C for 4 h to obtain purified glycolide crystals with an acid content of 72 µmol/g and a purity measured by GC of 96.8%.

### Example 8

Crude glycolide was prepared as in Example 7.

### Purification of glycolide:

The differences from Example 7 were only in that the melt from reactor A was added dropwise to reactor B at a rate of 190 wt% relative to the weight of the solid-liquid mixture per hour, and the internal temperature rose to a maximum of 14.8°C during the addition of the melt; after completion, a solid-liquid mixture with a solid content of 33.0 wt% was obtained. The median diameter of the glycolide crystals in the filter cake was 318 µm (the samples of the solid-liquid mixture were taken from the upper and lower parts of the reactor, the filter cake was collected by suction filtration, and the median diameter of the crystals in the filter cake was measured to have a deviation of 19.3%), the ratio of D90 to D10 was 4.5, the acid content of the purified glycolide crystals was 77 µmol/g, and the purity measured by GC was 96.5%;

Except for the above-mentioned differences, others were the same as those in Example 7.

### Example 9

Crude glycolide was prepared as in Example 7.

### Purification of glycolide:

The differences from Example 7 were only in that the melt from reactor A was added dropwise to reactor B at a rate of 120 wt% relative to the weight of the solid-liquid mixture per hour, and the internal temperature rises to a maximum of 13.5°C during the addition of the melt; after completion, a solid-liquid mixture with a solid content of 32.9 wt% was obtained. The median diameter of the glycolide crystals in the filter cake was 305 µm (the samples of the solid-liquid mixture were taken from the upper and lower parts of the reactor, the filter cake was collected by suction filtration, and the median diameter of the crystals in the filter cake was measured to have a deviation of 18.2%), the ratio of D90 to D10 was 4.2, the acid content of the purified glycolide crystals was 76 µmol/g, and the purity measured by GC was 96.6%;

Except for the above-mentioned differences, others were the same as those in Example 7.

### Example 10

Crude glycolide was prepared as in Example 7.

### Purification of glycolide:

The differences from Example 7 were only in that 250 g of crude glycolide solid was heated and melted, and the melt was maintained at 80°C in jacketed reactor A; after completion, a solid-liquid mixture with a solid content of 32.8 wt% was obtained, the median diameter of glycolide crystals in the filter cake was 290 µm (the samples of the solid-liquid mixture were taken from the upper and lower parts of the reactor, the filter cake was collected by suction filtration, and the median diameter of the crystals in the filter cake was measured to have a deviation of 16.7%), the ratio of D90 to D10 was 5.0, the acid content of the purified glycolide crystals was 70 µmol/g, and the purity measured by GC was 96.8%;

Except for the above-mentioned differences, others were the same as those in Example 7.

### Example 11

Crude glycolide was prepared as in Example 7.

### Purification of glycolide:

The differences from Example 7 were only in that 600 g of the solid-liquid mixture I of ethyl acetate and glycolide was added to jacketed reactor B, wherein the glycolide solid suspended in the mixture was in an amount of 140 g; after completion, a solid-liquid mixture with a solid content of 37.6 wt% was obtained, the median diameter of the glycolide crystals in the filter cake was 279 µm (the samples of the solid-liquid mixture were taken from the upper and lower parts of the reactor, the filter cake was collected by suction filtration, and the median diameter of the crystals in the filter cake was measured to have a deviation of 15.5%), the ratio of D90 to D10 was 3.7, the acid content of the purified glycolide crystals was 68 µmol/g, and the purity measured by GC was 96.8%;

Except for the above-mentioned differences, others were the same as those in Example 7.

### Example 12

The difference from Example 7 was that the crude glycolide was different;
The crude glycolide used in the present invention was glycolide prepared according to the method described in Example 1 of the invention patent CN107868075A (conventional purification method), with an acid content of 12.0 µmol/g and a glycolide GC purity of 98.9%;
Except for the above-mentioned difference, other purification steps were the same as those in Example 7;

After purification of glycolide, a solid-liquid mixture with a solid content of 35.3 wt% was obtained, the median diameter of glycolide crystals in the filter cake was 311 µm (the samples of the solid-liquid mixture were taken from the upper and lower parts of the reactor, the filter cake was collected by suction filtration, and the median diameter of the crystals in the filter cake was measured to have a deviation of 14.8%), the ratio of D90 to D10 was 3.7, the acid content of the purified glycolide crystals was 0.80 µmol/g, and the purity measured by GC was 99.7%.

### Preparation of polyglycolic acid:

Polyglycolic acid was prepared as in Example 5, except that 55 g of the purified glycolide from Example 12 was weighed and added to the reactor; the intrinsic viscosity of the polyglycolic acid was measured to be 1.67 dL/g.

### Example 13

Preparation of crude L-lactide: according to the oligomerization-depolymerization method described in Source 1 of invention patent CN106397388A, crude L-lactide was prepared using L-lactic acid as raw material, with an acid content of 275.0 µmol/g and a crude lactide GC purity of 94.5%;

### Purification of L-lactide:

An excess amount of the above-mentioned crude L-lactide solid (acid content: 275.0 µmol/g, GC purity: 94.5%) was added to 600 g of isopropanol (commercially available, dehydrated with 3A molecular sieve), and the mixture was thoroughly mixed at 35°C until dissolution equilibrium was achieved. The solid-liquid mixture was subjected to solid-liquid separation at 35°C to obtain a clear saturated solution. 32 g of lactide solid was added to 498 g of the saturated solution so as to obtain a solid-liquid mixture I.

250 g of the above-mentioned crude lactide melt was maintained at 100°C in jacketed reactor A. 530 g of the mixture of isopropanol and lactide was added to jacketed reactor B, in which about 30 g of glycolide solid (with a median diameter of 139 µm, a median diameter deviation of 8.9% measured by sampling from the upper and lower parts of the reactor) was suspended, and in which the acid content of the liquid phase was 16.0 µmol/g. The mixture was stirred at 120 rpm and maintained at 35°C. The melt from reactor A was added dropwise to reactor B at a rate of 70 wt% relative to the weight of the solid-liquid mixture per hour, during which the stirring rate was 120 rpm and the internal temperature was maintained at 37°C. After completion, a solid-liquid mixture with a solid content of 32.2 wt% was obtained. The filter cake was collected by suction filtration under nitrogen protection. The median diameter of the lactide crystals in the filter cake was measured to be 337 µm (the samples of the solid-liquid mixture were taken from the upper and lower parts of the reactor, the filter cake was collected by suction filtration, and the median diameter of the crystals in the filter cake was measured to have a deviation of 18.0%), the ratio of D90 to D10 was 4.6. The filter cake was vacuum-dried at 60°C for 4 h to obtain purified lactide crystals with an acid content of 15 µmol/g and a purity measured by GC of 98.9%.

### Preparation of polylactic acid:

55 g of the purified L-lactide from Example 13 was weighed and added to a reactor, 0.02% by weight of lactide of stannous isooctanoate (commercially available) and 0.1% by weight of lactide of initiator lauryl alcohol (commercially available) were weighed and added to the reactor, the reactor was evacuated and then replaced with nitrogen, the evacuation-nitrogen replacement operation was repeated once, and finally the reactor was evacuated again to make the pressure inside the reactor lower than 200 Pa. Then, the reactor was closed for reaction under stirring. The reactor temperature was first heated to 130°C, and then the temperature was increased at a heating rate of 4°C/min, and finally the temperature was maintained at 190°C, and the reaction was carried out for 60 min before discharging to obtain polylactic acid.

The intrinsic viscosity of the polylactic acid was measured to be 1.02 dL/g.

### Example 14

Preparation of crude ε-caprolactam: according to the reaction method, vapor phase Beckmann rearrangement of cyclohexanone oxime, described in Example 1 of invention patent CN101070298A and a simple distillation method, crude ε-caprolactam in molten state was obtained with a GC purity of 99.2%.

### Purification of ε-caprolactam:

An excess amount of the above-mentioned crude ε-caprolactam in molten state (GC purity: 99.2%) was added to 600 g of isopropyl ether (commercially available), and the mixture was thoroughly mixed at 25°C until dissolution equilibrium was achieved. The solid-liquid mixture was subjected to solid-liquid separation at 25°C to obtain a clear saturated solution. 30 g of the solid obtained by cooling the above-mentioned crude ε-caprolactam in molten state to room temperature and then crushing was added to 500 g of the saturated solution so as to obtain a solid-liquid mixture I.

250 g of the above-mentioned crude ε-caprolactam melt was maintained at 75°C in jacketed reactor A. 530 g of the mixture of isopropyl ether and ε-caprolactam was added to jacketed reactor B, in which about 30 g of ε-caprolactam solid (with a median diameter of 97 µm, a median diameter deviation of 6.5% measured by sampling from the upper and lower parts of the reactor) was suspended. The mixture was stirred at 120 rpm, and maintained at 25°C. The melt from reactor A was added dropwise to reactor B at a rate of 35 wt% relative to the weight of the solid-liquid mixture per hour, during which the stirring rate was 120 rpm and the internal temperature was maintained at 25°C. After completion, a solid-liquid mixture with a solid content of 35.3 wt% was obtained. The filter cake was collected by filtration under nitrogen protection. The median diameter of the ε-caprolactam crystals in the filter cake was measured to be 308 µm (the samples of the solid-liquid mixture were taken from the upper and lower parts of the reactor, the filter cake was collected by suction filtration, and the median diameter of the crystals in the filter cake was measured to have a deviation of 12.9%), and the ratio of D90 to D10 was 4.2. The filter cake was vacuum-dried at 50°C for 4 h to obtain purified ε-caprolactam crystals with a purity measured by GC of 99.8%.

### Example 15

Preparation of crude trioxane: the trioxane raw gas from the top of the distillation column of a trioxane synthesis unit (overhead operating temperature: 117°C) was condensed to 75°C to obtain crude trioxane in molten state with a purity of 98.5%.

### Purification of trioxane:

An excess amount of the above-mentioned crude trioxane in molten state (GC purity: 98.5%) was added to 600 g of benzene (commercially available), and the mixture was thoroughly mixed at 8°C until dissolution equilibrium was achieved. The solid-liquid mixture was subjected to solid-liquid separation at 8°C to obtain a clear saturated solution. 30 g of the solid obtained by cooling the above-mentioned crude trioxane in molten state to room temperature and then crushing was added to 500 g of the saturated solution so as to obtain a solid-liquid mixture I.

250 g of the above-mentioned crude trioxane melt was maintained at 66°C in jacketed reactor A. 530 g of the mixture of benzene and trioxane was added to jacketed reactor B, in which about 30 g of trioxane solid (with a median diameter of 75 µm, a median diameter deviation of 6.3% measured by sampling from the upper and lower parts of the reactor) was suspended. The mixture was stirred at 120 rpm, and maintained at 8°C. The melt from reactor A was added dropwise to reactor B at a rate of 35 wt% relative to the weight of the solid-liquid mixture per hour, during which the stirring rate was 120 rpm and the internal temperature was maintained at 8°C. After completion, a solid-liquid mixture with a solid content of 32.7 wt% was obtained. The filter cake was collected by suction filtration under nitrogen protection. The median diameter of the trioxane crystals in the filter cake was measured to be 242 µm (the samples of the solid-liquid mixture were taken from the upper and lower parts of the reactor, the filter cake was collected by suction filtration, and the median diameter of the crystals in the filter cake was measured to have a deviation of 9.5%), and the ratio of D90 to D10 was 3.9. The filter cake was vacuum-dried at 55°C for 4 h to obtain purified trioxane crystals with a purity measured by GC of 99.7%.

### Comparative Example 1

### Preparation of crude glycolide:

Crude glycolide was prepared as in Example 1, with an acid content of 450 µmol/g and a glycolide purity of 90.20%.

### Purification of glycolide:

250 g of the above-mentioned crude glycolide melt was maintained at 90°C in jacketed reactor A. 500 g of ethyl acetate was added to jacketed reactor B, stirred at 120 rpm, and maintained at 10°C. The melt from reactor A was transferred to reactor B at a dropwise addition rate of 70 wt% relative to the weight of the solvent per hour, during which the stirring rate was 120 rpm and the internal temperature was maintained at 12°C. After completion, a solid-liquid mixture with a solid content of 25.1 wt% was obtained. The filter cake was collected by suction filtration under nitrogen protection. The median diameter of the glycolide crystals in the filter cake was measured to be 725 µm (the samples of the solid-liquid mixture were taken from the upper and lower parts of the reactor, the filter cake was collected by suction filtration, and the median diameter of the crystals in the filter cake was measured to have a deviation of 31.3%), the ratio of D90 to D10 was 11.1. The filter cake was vacuum-dried at 60°C for 4 h to obtain purified glycolide crystals with an acid content of 123 µmol/g and a purity measured by GC of 96.5%.

### Comparative Example 2

The purified glycolide crystals (GC purity: 96.5%) obtained in Comparative Example 1 were subjected to secondary purification.

### Secondary purification of glycolide:

150 g of the purified glycolide crystals obtained in Comparative Example 1 were heated and melted, and the melt was maintained at 90°C in jacketed reactor A. 300 g of ethyl acetate was added to jacketed reactor B, stirred at 120 rpm, and maintained at 10°C. The melt from reactor A was transferred to reactor B at a dropwise addition rate of 70 wt% relative to the weight of the solvent per hour, during which the stirring rate was 120 rpm and the internal temperature was maintained at 12°C. After completion, a solid-liquid mixture with a solid content of 26.9 wt% was obtained. The filter cake was collected by suction filtration under nitrogen protection. The median diameter of the glycolide crystals in the filter cake was measured to be 843 µm (the samples of the solid-liquid mixture were taken from the upper and lower parts of the reactor, the filter cake was collected by suction filtration, and the median diameter of the crystals in the filter cake was measured to have a deviation of 34.7%), the ratio of D90 to D10 was 8.5. The filter cake was vacuum-dried at 60°C for 4 h to obtain purified glycolide crystals with an acid content of 18.1 µmol/g and a purity measured by GC of 98.5%.

### Preparation of polyglycolic acid:

Polyglycolic acid was prepared as in Example 5, except that 55 g of the purified glycolide from Comparative Example 2 was weighed and added to the reactor; the intrinsic viscosity of the polyglycolic acid was measured to be 0.95 dL/g.

### Comparative Example 3

### Preparation of crude glycolide:

Crude glycolide was prepared as in Example 1, with an acid content of 450 µmol/g and a glycolide purity of 90.20%.

### Purification of glycolide:

250 g of the above-mentioned crude glycolide melt was maintained at 90°C in jacketed reactor A. 500 g of ethyl acetate was added to jacketed reactor A, stirred at 120 rpm, and maintained at 60°C for 30 min to prepare a solution. The solution in reactor A was cooled to an internal temperature of 12°C at a rate of 12°C per hour, during which the stirring rate was 120 rpm; the internal temperature of the solid-liquid mixture was maintained at 12°C for 30 min to allow the crystals to fully precipitate. After completion, a solid-liquid mixture with a solid content of 23.6 wt% was obtained. The filter cake was collected by suction filtration under nitrogen protection. The median diameter of the glycolide crystals in the filter cake was measured to be 692 µm (the samples of the solid-liquid mixture were taken from the upper and lower parts of the reactor, the filter cake was collected by suction filtration, and the median diameter of the crystals in the filter cake was measured to have a deviation of 28.2%), and the ratio of D90 to D10 was 10.7. The filter cake was vacuum-dried at 60°C for 4 h to obtain purified glycolide crystals with an acid content of 108 µmol/g and a purity measured by GC of 96.9%.

In Comparative Example 1, glycolide was purified by a conventional method. By comparing Example 1 with Comparative Example 1, it can be seen that the purified solid phase had the median diameter of 275 µm and 725 µm, the acid content of 45 µmol/g and 123 µmol/g, the ratio of D90 to D10 of 3.9 and 11.1, and the GC purity of 98.0% and 96.5%, respectively;

In Comparative Example 2, the glycolide obtained in Comparative Example 1 was subjected to secondary purification using a conventional method. In Example 5, the glycolide obtained in Example 1 was subjected to secondary purification using the method of the present invention. By comparing Example 5 with Comparative Example 2, it can be seen that the purified solid phase had the median diameter of 295 µm and 843 µm, the acid content of 2.8 µmol/g and 18.1 µmol/g, the ratio of D90 to D10 of 3.6 and 8.5, and the GC purity of 99.6% and 98.5%, respectively;

From the comparison of above data, it can be seen that compared with the conventional purification method in the prior art, the purification method of the present invention results in purified solid phase with smaller median diameter, lower acid content, more uniform particle diameter distribution, and higher purity. The method of the present invention can be used for secondary purification, and the glycolide obtained after secondary purification exhibits better properties.

In Example 12, the glycolide with a GC purity of 98.9% obtained by a conventional purification method was further purified. The median diameter of the obtained glycolide was 311 µm, the ratio of D90 to D10 was 3.7, the acid content of the purified glycolide crystals was 0.80 µmol/g, and the purity measured by GC was 99.7%. This further demonstrates that the present invention can be used to purify glycolide with a higher purity, resulting in reduced acid content and also improved purity.

The test results demonstrate that the glycolide purified in Examples 1-12 exhibits smaller and uniform particle diameter, low acid content, and high purity. In addition, the present invention uses a solid-liquid mixture to receive the glycolide melt. And according to the present invention, a solid-liquid mixture is used to receive glycolide melt. Compared with the traditional recrystallization process, the present invention enables a constant process temperature, no cooling process with a large temperature span, simple operation, low energy consumption, and is suitable for industrialization.

## Claims

1. A phase transition-diffusion coupled crystallization method for purifying a crystalline substance, comprising the following steps:
(1) adding a crude crystalline substance in molten state to a solid-liquid mixture I in dissolution equilibrium state to obtain a solid-liquid mixture II; wherein the solid-liquid mixtures I and II each comprise a solid phase and a solution phase, the solid phase comprises the crystalline substance in solid state and possible impurities, and the solution phase comprises a solvent, the crystalline substance in dissolved state and possible impurities; and
(2) subjecting the solid-liquid mixture II to a solid-liquid separation.

2. The method according to claim 1, wherein the crude crystalline substance in molten state has a temperature higher than that of the solid-liquid mixture **I,** and the temperature difference thereof is sufficient to cause the crude crystalline substance in molten state to undergo a phase transition and complete the phase transition.

3. The method according to claim 1 or 2, wherein the crystalline substance in the solid phase of the solid-liquid mixture I has a purity higher than that of the crude crystalline substance in molten state, and the purity difference thereof is sufficient to cause an effective amount of impurities in the crude crystalline substance in molten state to diffuse into the solvent.

4. The method according to any one of the preceding claims, **characterized in that**:
in step (1), the crude crystalline substance in molten state is added to the solid-liquid mixture I in dissolution equilibrium state in a manner and at a rate such that the temperature during the transformation of the solid-liquid mixture I to the solid-liquid mixture II remains unchanged or substantially unchanged, or changes by no more than 10°C, preferably by no more than 5°C, and more preferably by no more than 3°C;
preferably, the crude crystalline substance in molten state is added to the solid-liquid mixture II in a manner selected from at least one of dropwise addition, injection, spraying, sprinkling, atomization.

5. The method according to claim 4, **characterized in that** an equipment containing the solid-liquid mixture I in step (1) is equipped with a heat exchange device to remove the heat brought by the addition of the crude crystalline substance in molten state, such as a jacket or a coil, in which a cooling medium such as water is used.

6. The method according to any one of the preceding claims, **characterized in that** the temperature difference between the crude crystalline substance in molten state and the solid-liquid mixture I and/or the solid-liquid mixture II is more than or equal to 15°C, preferably more than or equal to 25°C; and less than or equal to 85°C, for example less than or equal to 75°C.

7. The method according to any one of the preceding claims, **characterized in that** the temperature of the solid-liquid mixture I and/or the solid-liquid mixture II is more than or equal to 4°C.

8. The method according to any one of the preceding claims, **characterized in that** the temperature of the crude crystalline substance in molten state is higher than or equal to the melting point of the crystalline substance; preferably, the temperature of the crude crystalline substance in molten state is at least 1°C, preferably at least 3°C higher than the melting point of the crystalline substance, and/or preferably, the temperature of the crude crystalline substance in molten state is no more than 15°C, preferably no more than 10°C higher than the melting point of the crystalline substance.

9. The method according to any one of the preceding claims, **characterized in that** the temperature of the crude crystalline substance in molten state is lower than or equal to the melting point of the crystalline substance; preferably, the temperature of the crude crystalline substance in molten state is at least 1°C, preferably at least 3°C lower than the melting point of the crystalline substance, and/or preferably, the temperature of the crude crystalline substance in molten state is no more than 15°C, preferably no more than 10°C lower than the melting point of the crystalline substance.

10. The method according to any one of the preceding claims, **characterized in that** the solid-liquid mixture I and/or the solid-liquid mixture II is a suspension;
preferably, the solid phase in the solid-liquid mixtures I and II is uniformly dispersed, and when two different samples of the solid-liquid mixture are taken from any two different positions in the solid-liquid mixture I to measure D50 particle diameter, the deviation in D50 particle diameter between the two different samples of the solid-liquid mixture is no more than 30%, preferably no more than 20%; and/or, when two different samples of the solid-liquid mixture are taken from any two different positions in the solid-liquid mixture II to measure D50 particle diameter, the deviation in D50 particle diameter between the two different samples of the solid-liquid mixture is no more than 30%, preferably no more than 20%.

11. The method according to any one of the preceding claims, **characterized in that** in the solid phase of the solid-liquid mixture I and/or the solid-liquid mixture II, the ratio of D90 to D10 of the solid phase is respectively no more than 10, preferably no more than 7, and more preferably no more than 5.

12. The method according to any one of the preceding claims, wherein the crystalline substance is a cyclic monomer with a melting point higher than -20°C, preferably selected from lactones (such as caprolactone, glycolide, lactide, 1,4-dioxane-2-one), cyclic formals (such as trioxane), and lactams (such as caprolactam), preferably lactide compounds, such as glycolide or lactide.

13. The method according to any one of the preceding claims, wherein the crystalline substance is glycolide; the crude crystalline substance in molten state is unpurified crude glycolide in molten state and/or purified crude glycolide in molten state; and/or,
the mass content of glycolide in the crude glycolide in molten state is 85%-99%.

14. The method according to claim 13, **characterized in that**:
the temperature of the unpurified crude glycolide in molten state is 75°C-120°C, preferably 80°C-95°C; and/or,
the temperature of the purified crude glycolide in molten state is 80°C-120°C, preferably 85°C-95°C.

15. The method according to claim 13, **characterized in that**:
in step (1),
the crude glycolide in molten state is added at a rate of 20 wt%-500 wt%, preferably 50 wt%-200 wt% relative to the weight of the solid-liquid mixture I per hour; and/or,
the temperatures of both the solid-liquid mixture I and the solid-liquid mixture II are not higher than 60°C; preferably, not higher than 50°C.

16. The method according to claim 13, **characterized in that**:
in step (1),
the solid phase of the solid-liquid mixture I is glycolide crystals with a glycolide purity higher than 95%; preferably glycolide crystals with a glycolide purity higher than 99%; and/or,
the liquid phase of the solid-liquid mixture I is a saturated solution of glycolide in an organic solvent; the organic solvent is preferably at least one of acetone, ethyl acetate, ethanol, n-propanol, iso-propanol, n-butanol, iso-butanol, tert-butanol, n-pentanol and iso-pentanol.

17. The method according to any one of the preceding claims, **characterized in that**:
in step (1),
the weight content of the solid phase in the solid-liquid mixture I is 0.5%-25%; preferably 2%-25%; and/or,
the weight content of the solid phase in the solid-liquid mixture II is less than or equal to 50%, preferably less than or equal to 40%.

18. The method according to any one of the preceding claims, **characterized in that**:
in step (1),
the median diameter of the solid phase in the solid-liquid mixture I is less than or equal to 200 microns; preferably less than or equal to 150 microns; and/or,
the median diameter of the solid phase in the solid-liquid mixture II is less than or equal to 600 microns; preferably less than or equal to 500 microns.

19. The method according to any one of the preceding claims, **characterized in that**:
the solid-liquid separation method in step (2) is centrifugation or filtration; and/or,
the method further comprises step (3): drying the solid phase separated and subsequently recovered in step (2), wherein the drying method is convection drying under hot inert gas or thermal conduction drying under vacuum.

20. The method according to any one of the preceding claims, wherein the method is implemented repeatedly, for example for 2, 3, 4 or 5 times; wherein, the purified crystalline substance obtained from the previous implementation of the method is melted to serve as the crude crystalline substance in molten state for the next implementation of the method.

21. Use of glycolide prepared by the method according to any one of claims 13-16 in the preparation of polyglycolic acid.
